# EUROPÄISCHE PATENTSCHRIFT

(11) **EP 2 338 564 B1**
(45) Veröffentlichungstag und Bekanntmachung des Hinweises auf die Patenterteilung: **27.03.2013**
(21) Anmeldenummer: 10192899.2
(22) Anmeldetag: 29.11.2010
(51) Int. Cl.: A61N 1/37

(54) **MRT-Optokoppler**
MRI optocoupler
Optocoupleur IRM

(30) Priorität: 22.12.2009 US 288856 P
(43) Veröffentlichungstag der Anmeldung: 29.06.2011
(73) Patentinhaber: BIOTRONIK CRM Patent AG, 6341 Baar (CH)
(72) Erfinder: Dörr, Thomas, 12437, Berlin (DE); Weiss, Ingo, 12435, Berlin (DE)
(74) Vertreter: Lindner-Vogt, Karin L.

(56) Entgegenhaltungen:
- US-A1- 2003 195 570
- US-A1- 2004 088 012
- US-A1- 2005 043 761
- US-A1- 2008 208 276

## Beschreibung

Die Erfindung bezieht sich auf eine Vorrichtung und ein Verfahren zur Erkennung von elektromagnetischen Feldern, speziell solche Felder, wie sie bei bildgebenden Kernspintomographieuntersuchungen (im folgenden MRT oder MRI) auftreten.

Obwohl MRI-Untersuchungen einen immer höheren Stellenwert in der diagnostischen Medizin erhalten, ist ein Teil der Patienten für MRI-Untersuchungen kontraindiziert. Eine solche Kontraindikation kann durch ein aktives implantiertes medizinisches Gerät (im Folgenden auch Implantat oder IMD) gegeben sein. Neben den MRI-Untersuchungen stellen aber auch andere technische Anwendungen eine Gefahr für die Nutzer von medizinischen Geräten oder implantierbaren medizinischen Geräten dar, besonders wenn diese starke elektromagnetische Störfelder (EMI, Electro Magnetic Interference) in Ihrer Umgebung erzeugen.

Um MRI-Untersuchungen dennoch zu ermöglichen sind verschiedene Ansätze, die sich entweder auf die Durchführung der MRI-Untersuchung oder auf das implantierbare medizinische Gerät beziehen, bekannt.

Unter anderem sind Technologien zur Erkennung von Magnetfeldern bekannt, die auf herkömmlichen Verfahren zur Magnetfelddetektion beruhen. So beschreibt die US 2008/0154342 ein Verfahren unter Ausnutzung eines GMR-Sensors (Giant Magnetic Resistance), um problematische Magnetfelder von MRT-Geräten zu erkennen. Diese technologischen Ansätze sind allerdings wenig spezifisch und erzeugen einen erhöhten Energiebedarf, was zu einer kürzen Betriebsdauer bei gleichen Energiereserven führt.

Ein weiterer technologischer Ansatz ist das Verwenden von optischer Signalübertragung anstelle der typischen Elektrodenleitungen, die auf der elektrischen Signalübertragung beruhen. Durch die Verwendung dieser optischen Leitungen verhindert man das Einkoppeln der elektromagnetischen Störfelder eines MRI-Gerätes in die Elektrodenleitungen, aber das Gesamtsystem wird komplexer, da einerseits die elektrischen Signale erst in optische Signale umgewandelt werden müssen und aus den optischen Signalen am Stimulationsort an Hand der Signale die Stimulationsimpulse generiert werden müssen und andererseits am Stimulationsort gemessene Signale ebenfalles umgewandelt werden müssen. Solche komplexeren Systeme erhöhen in der Regel auch den Energiebedarf eines Implantats. Ein solches System, dass auf optischer Signalleitung basiert ist in der US 2005/0090886 und der US 7,450,996 beschrieben.

Die US2008/0208276 beschreibt ein Verfahren, bei dem mittels eines Widerstandes die induzierten Ströme gemessen werden und ein optisches Signal zur Datenübermittlung generiert wird und die Daten optisch aus dem Bereich geleitet werden, der im Wirkungsradius eines MRT-Gerätes liegt.

Aufgabe der vorliegenden Erfindung ist es, ein implantierbares medizinisches Gerät bereitzustellen, das eine zuverlässige und sichere Erkennung von MRT-Störfeldern erlaubt.

### Zusammenfassung der Erfindung

Die Aufgabe wird durch ein implantierbares medizinisches Gerät (IMD) mit den Merkmalen des Anspruchs 1 gelöst.

Das implantierbare medizinische Gerät (IMD) beinhaltet dabei mindestens ein hermetisch abgeschlossenes Gehäuse, mindestens einer Steuereinheit, mindestens eine MRT-Störfelderkennungseinheit, die mit mindestens der einen Steuereinheit und mindestens einer Elektrode und/oder mindestens einer Antenne und/oder mindestens einer Spule verbunden oder verbindbar ist, wobei die MRT-Störerkennungseinheit mindestens einem elektrooptischen Wandler besitzt, der induzierte Spannungen aus der mindestens einen Elektrode und/oder der mindestens einen Antenne und/oder der mindestens einen Spule in optische Signale umwandelt und diese innerhalb der MRT-Störfelderkennungseinheit an eine Auswerteeinheit der MRT-Störfelderkennungseinheit optisch und potentialfrei überträgt und die Auswerteeinheit bei überschreiten einer Schwelle der optischen signale und/oder einem vorbestimmten periodischen Auftreten der optischen Signale ein Umschalten in einen MRI-sicheren Zustand auslöst.

Die MRT-Störfelderkennungseinheit kann dabei auch modular ausgeführt sein, das heißt, dass zum Beispiel, aber nicht beschränkt auf, der elektrooptische Wandler auch räumlich getrennt von dem Rest der MRT-Störfelderkennungseinheit lokalisiert sein kann und die Lichtsignale über optische Leiter, wie, aber nicht beschränkt auf Glasfasern, zu der MRT-Störerkennungseinheit übertragen werden. Auch können andere Komponenten der MRT-Störerkennungseinheit räumlich von dieser getrennt sein, wobei eine optische Signalleitung gegenüber der elektrischen Signalleitung über längere Strecken bevorzugt wird.

Auch ist bevorzugt, dass elektrooptische Wandler auf unterschiedlichen Frequenzen emittieren, speziell bei verschieden Empfängern können mehrere Emissionsfrequenzen verwendet werden, auch um nach dem Entstehungsort der induzierten Spannungen differenzieren zu können und diesen in die Auswertung einfließen lassen zu können. Dadurch sind auch differenzierte Reaktionen auf das Auftreten von MRT-Störungen möglich, je nachdem welche Elemente des IMD und/oder der zugehörigen Elektrodenleitungen und/oder Elektroden betroffen sind.

Des Weiteren wird bevorzugt, dass die Erkennungseinheit für MRT-Störfelder über mindestens einen Sensor verfügt, der die optischen Signale von dem mindestens einen elektrooptischen Wandler in elektrische Signale umwandelt, die zur MRT-Erkennung verwendet werden, wobei auch die Signale von mehreren elektrooptischen Wandlern zu einem Sensor führen können.

Ebenfalls bevorzugt ist, dass mindestens eine Elektrode vorhanden ist, die therapeutischen und/oder diagnostischen Zwecken dient, wobei die Elektrode sich außerhalb der des hermetisch verschlossen Gehäuses erstrecken kann oder sich auf der Außenfläche diese Gehäuses befinden kann oder Teil dieses Gehäuses sein kann.

In einer weiteren bevorzugten Ausführungsform ist die mindestens eine Elektrode eine Verbindung zu mindestens einem, vom Implantat ausgehenden distal angeordneten Sensor und/oder mindestens einem Aktuator.

Auch ist bevorzugt, dass der elektrooptische Wandler ein Optokoppler ist.

Besonders bevorzugt ist, dass der elektrooptische Wandler ein auf dem Kerreffekt basierender Sensor für elektromagnetische Felder ist.

Des Weiteren ist bevorzugt, dass der elektrooptische Wandler ein Sensor für elektromagnetische Felder ist, der auf den von externen elektromagnetischen Feldern abhängigen optischen Eigenschaften eines Kristalls basiert. Ein Beispiel für ein System, das sich als ein solcher Sensor eignet, findet sich in LiTaO3 -> HIGH-RESOLUTION ELECTRO-OPTIC MAPPING OF NEAR-FIELD, DISTRIBUTIONS IN INTEGRATED MICROWAVE CIRCUITS, K. Yang, G. David, S. Robertson, J.F. Whitaker, and L.P.B. Katehi, Microwave Symposium Digest, 1998 IEEE MTT-S International Volume 2, Issue , 7-12 Jun 1998 Page(s):949 - 952 vol.2 Digital Object Identifier 10.1109/MWSYM.1998.705148.

Auch wird bevorzugt, dass sich der elektrooptische Wandler innerhalb oder außerhalb oder in einer Gehäusedurchführung des hermetisch abgeschlossenen Gehäuses liegt.

Ebenfalls bevorzugt ist, dass der elektrooptische Wandler nur Informationen zu den Amplituden und/oder der Einhüllende der Amplituden und/oder den Frequenzen und/oder bei mehren elektrooptischen Wandlern Phasen überträgt.

Besonders bevorzugt ist, dass die Erkennungseinheit für MRT-Störfelder aus dem optischen Signal einen skalaren Wert ermittelt, der die identifizierte Feldstärke repräsentiert und bei Überschreiten eines voreinstellbaren und/oder variablen Schwellwertes oder bei Erreichen eines voreinstellbaren und/oder variablen Schwellwertbereichs ein MRT-Feld erkennt, wobei der skalare Wert auch eine funktionale oder logische Verknüpfung aus einer oder mehreren der Informationen, wie Amplitudeninformation und/oder Frequenzinformation und/oder Phaseninformation, oder der gewichteten Informationen sein kann.

In einer weiteren bevorzugten Ausführungsform erfolgt die Gewichtung der Informationen über eine vorbestimmte Dämpfung der optischen Signale bei der Übertragung der optischen Signale, wobei zusätzlich unterschiedliche Dämpfungen für unterschiedlichen Signalquellen oder Informationsarten über verschiedene Übertragungsfrequenzen realisiert werden können.

Des Weiteren ist bevorzugt, dass mehrere elektrische Zuleitungen zum elektrooptischen Wandler in Reihe und/oder parallel geschaltet sind.

Auch wird bevorzugt, dass der elektrooptische Wandler mit mindestens einem Schutzelement in Reihe und/oder parallel geschaltet ist. Als Schutzelemente sind zum Beispiel, aber nicht ausschließlich, EMI-Kondensatoren und/oder Schutzdioden zu verstehen.

Ebenfalls wird bevorzugt, dass der optische Wandler zwischen zwei Zuleitungen geschaltet ist, wobei Zuleitungen neben elektrischen Leitungen auch flächige Elektroden wie das Implantatgehäuse umfasst.

Bevorzugt ist auch, dass der Schwellwert zur MRT-Erkennung eine gewichtete Funktion der ermittelten Frequenz der induzierten Spannungen ist und/oder der Schwellwert und/oder die Gewichtungsfaktoren eine Funktion von mit anderen Sensoren oder Indikatoren bestimmten statischen Feldstärke ist oder sind, wobei die funktionale Abhängigkeit sowohl linear als auch nicht linear sein kann.

Des Weiteren wird bevorzugt, dass der durch die MRT-Störerkennungseinheit einschaltete oder ausgelöste MRT-sichere Zustand für eine vorbestimmte oder vorbestimmbare Zeit eingeschaltet und nach dem Ablauf der Zeit deaktiviert oder eine weitere MRT-Erkennung vorgenommen.

Auch wird bevorzugt, dass eine MRT-Erkennung nur stattfindet, wenn zeitgleich mit der MRT-Erkennung nach Anspruch 1 auch mindestens eine weitere Messmethode eine MRT-Erkennung signalisiert, wobei unter weiteren Methoden unter anderem, aber nicht ausschließlich, folgende Methoden zu verstehen sind, GMR-Sensoren, MagFET-Sensoren, Hallsensoren, die Überwachung von Batteriespannungen während Kondensatorladeprozessen, die Detektion von RF-Feldern, die Detektion von magnetischen Grandientenfeldern, die Detektion von durch elektromagnetische Felder induzierten Strömen, die Detektion von spezifischen Vibrationen oder als Sensoren ausgelegte Bauteile zur Detektion von durch Lorentzkräfte induzierten Vibrationen.

Ebenfalls wird bevorzugt, dass mindestens eine der folgenden Maßnahmen bei einer MRT-Erkennung oder durch das MRT-Erkennungssignal eingeleitet werden, das Wechseln in einen MRI-sicheren Zustand, ein verlängertes Verbleiben in einem MRI-sicheren oder gegenüber elektromagnetischen Störfeldern unempfindlichen Zustand, eine Synchronisierung von elektrischen Messungen (z. B. Impedanzmessungen) mit bei periodischen oder gepulsten elektromagnetischen Feldern auftretenden betragsmäßigen Feldstärkenminima oder die Synchronisierung einer Stimulation mit eben jenen betragsmäßigen Minima, und die Abgabe von elektromagnetischen Pulsen zur Signalisierung, dass ein medizinisches Gerät, speziell ein Implantat, im elektromagnetischen Feld vorhanden ist, speziell zur Signalisierung an ein MRI-Gerät, mit der Möglichkeit neben der Störung auch Informationen auf diese Weise zu übertragen und auf dem Bildschirm des MRI sichtbar zu machen.

Des Weiteren wird bevorzugt, dass ein Lagesensor zur Plausibilitätsprüfung herangezogen wird und eine positive MRI-Erkennung nur erfolgt, wenn der Lagesensor eine liegende Positur und/oder eine andere voreinstellbare Positur meldet.

Besonders bevorzugt ist der Lagesensor selbst kalibrierend, wobei die Kalibrierung unter voreinstellbaren Randbedingungen, wie, aber nicht beschränkt auf, Uhrzeiten und/oder Herzraten und/oder Atemfrequenz und/oder hämodynamische Parameter und/oder Aktivität (Bewegungssensor) stattfindet.

### Beschreibung der Figuren:

Einige Aspekte der Erfindung werden in den Figuren 1 -6 dargestellt.
- Fig. 1: Schematische Darstellung des Ablaufs einer MRI-Untersuchung
- Fig. 2: Blockschaltbild eines erfindungsgemäßen Implantats mit einem elektrooptischen Wandler zur Detektion von elektromagnetischen Störfeldern
- Fig. 3: Blockschaltbild eines erfindungsgemäßen Implantats mit einem elektrooptischen Wandler zur Detektion von elektromagnetischen Störfeldern
- Fig. 4: Blockschaltbild einer erfindungsgemäßen Eingangsschutzschaltung
- Fig. 5: Blockschaltbild einer erfindungsgemäßen Eingangsschutzschaltung mit einem Gewichtungsfilter
- Fig. 6: Blockschaltbild einer erfindungsgemäßen komplexen Eingangsschutzschaltung

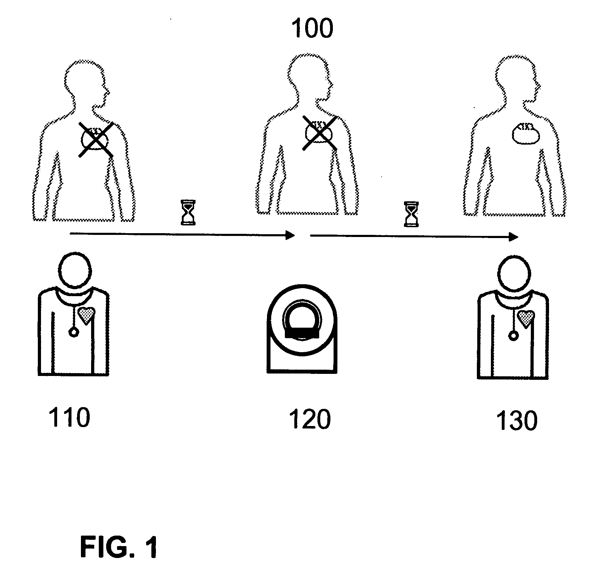

Figur 1 beschreibt den Stand der Technik, bei dem der ICD-Patient (100) vor der geplanten MRT-Untersuchung von einem Kardiologen nachgesorgt und der ICD ausgeschaltet (110) wird. Mit einer zeitlichen Verzögerung von Stunden bis Tagen erfolgt die MRT-Untersuchung bei einem Radiologen (120). Nach einer weiteren Verzögerung wird der Patient wieder vom Kardiologen (130) betreut und der ICD wieder eingeschaltet. Während der gesamten Zeit von (110) bis (130) ist der Patient ohne den Schutz des implantierten Defibrillators und weitgehend ohne Rhythmusmonitoring. Derzeit wird dieses verbleibende Restrisiko, gemessen an dem Nutzen der MRT-Untersuchung in Kauf genommen.

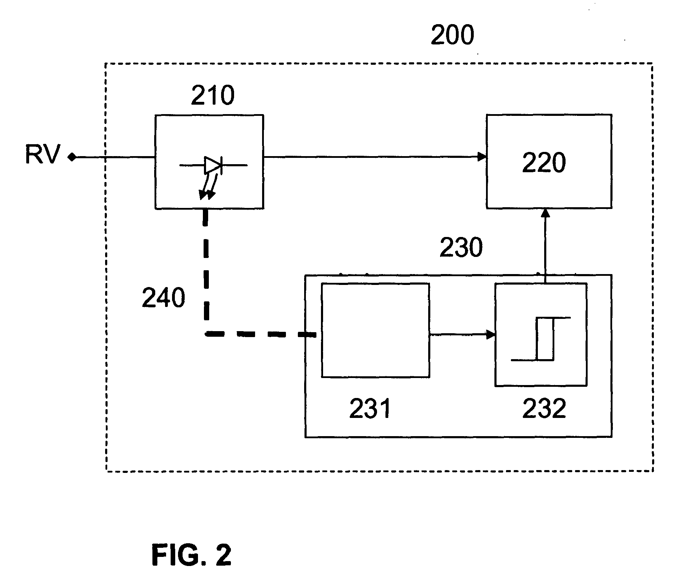

Figur 2 zeigt ein Blockschaltbild der erfindungsgemäßen Lösung zum Detektieren von starken Magnetfeldern, insbesondere von variablen elektromagnetischen Feldern. Dieses Ausführungsbeispiel bezieht sich der Einfachheit halber auf einen Einkammerherzschrittmacher. Eine Ausführung als Mehrkammerschrittmacher, ICD, CRT-Gerät, Medikamentenpumpe, Überwachungsgerät für physiologische Körperfunktionen und/oder Neurostimulator ist für einen Fachmann einfache Variation und einfach möglich. Im Folgenden bezieht sich die Formulierung Implantat auf ein erfindungsgemäßes implantierbares medizinisches Gerät. Die Elektrodenleitung (RV) gelangt dafür durch eine Durchführung in das Innere des Implantates (200). Im Implantat (200) werden die elektrischen Signale über einen elektrooptischen Wandler an die Implantatselektronik weitergeleitet. Sind elektromagnetische Störfelder einer vorbestimmbaren Intensität vorhanden, so emittiert der elektrooptische Wandler licht, das über den Lichtleiter oder optischen Link (240) an eine Steuereinheit (230) weiterleitet. Die Steuereinheit (230) beinhaltet mindestens einen optischen Sensor (231), der mit dem optischen Leiter verbunden ist und einen Schwellwertkomparator (232), der wiederum mit dem optischen Sensor (231) verbunden ist. Der Schwellwertkomparator (232) vergleicht das Signal des optischen Sensors (231) mit einem oder mehreren voreinstellbaren Schwellwerten und gibt als Ergebnis des Vergleichs entweder ein Signal an die Implantatselektronik (220), ob ein oder mehrere voreinstellbare Schwellwerte überschritten wurde und/oder ein Signal, das angibt um wie viel ein voreinstellbarer Schwellwert überschritten wurde. Als Reaktion des Implantatselektronik (220) auf das Signal der Steuereinheit (230), genauer des Schwellwertkomparators (232), ändert die Implantatselektronik (220) einen voreinstellbaren Betriebsmodus, der einen unproblematischen Betrieb des Implantats unter den gegebenen elektromagnetischen Einflüssen erlaubt.

Eine weitere mögliche Ausführung ist, dass das MRT-RF-Feld oder eine andere elektromagnetische Störung eine LED, die an der Durchführung des Implantates oder an einer anderen Stelle innerhalb oder außerhalb des Implantates angebracht ist, zum emittieren von optisch detektierbarer elektromagnetischer Strahlung anregt. Vereinfacht ausgedrückt heißt das, dass wenn es leuchtet, dann ist ein MRT oder ein anderes starkes elektromagnetischen Feld mit einem Gefahrenpotential ähnlich dem eines MRT in der Umgebung des Implantates (200) vorhanden. Diese LED kann entweder Bestandteil eines Optokopplers (210) oder der ohnehin benötigten Protection- (Schutz-) Transistoren (Arrays) sein und immer dann die Elektroden und das Gehäuse von der Implantatselektronik (220) entkoppeln, wenn die LED leuchtet. Von besonderem Nutzen ist diese Lösung für Implantate, die keine oder unzureichende gefilterte Durchführungen besitzen. Sind die gefilterten Durchführungen zum Schutz der Elektronik vor hohen RF-Spannungen zwar ausreichend, kann diese LED im Implantatsheader, das heißt im Anschlusssegment des Implantats, meist aus Kunststoff mit den Anschlüssen, die die Durchführungen des Implantats (200) mit den Elektroden (RV) verbinden, (oder weiter distal) montiert sein und die Information über eine optische Durchführung störungsfrei ins Innere gebracht werden um dort Schutzmechanismen gegen anderen elektromagnetischen Felder zu schalten.

Vorteil ist die bezugspotentialfreie RF-Detektion, woraus die fehlende Gefahr der Demodulation im Implantat resultiert. So können auch die EMI-Kondensatoren entfallen und die Anforderungen von HDS (hämodynamischer Sensor) und MRT in einem ICD/IPG kombiniert werden.

Auch ermöglicht eine Ausführung, das Elektrodeninterface im Falle einer vorliegenden RF-Störung im MRT oder durch andere elektromagnetischen Störquellen abzuschalten, wobei die verbleibende Einkopplung der RF-Störung in das Innere des Implantates extrem minimiert wird. Erfindungsgemäß wird die elektrische Komponente des Störfeldes zu dessen Identifikation genutzt, im Gegensatz zu US 7,164,950 B2 wo die induktive Wahrnehmung der hochfrequenten Störfelder innerhalb eines gut leitfähigen (z. B. metallischen) Gehäuses nicht möglich wäre.

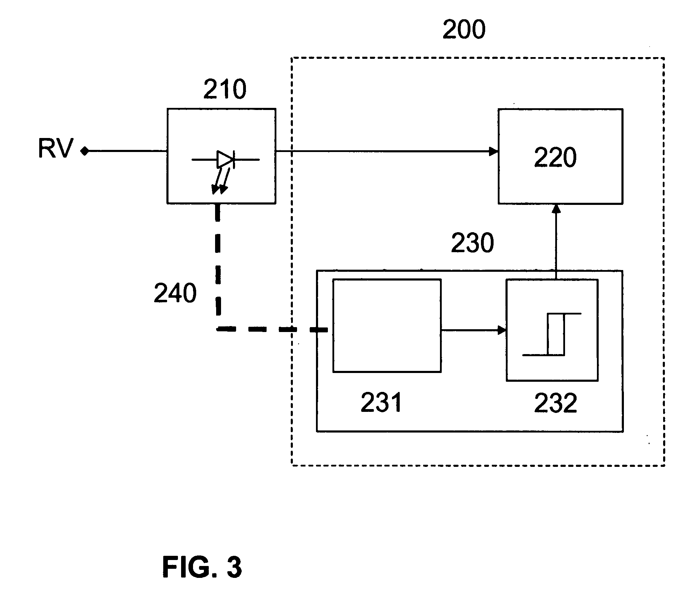

Die Figur 3 zeigt eine Ausführungsform, bei der sich der elektrooptische Wandler (210) außerhalb des Implantates (200) befindet. Die Ausführung ist ansonsten analog zu der in Figur 2 ausgeführt, allerdings ist es aufgrund der Lage des elektrooptischen Wandlers (210) notwendig, dass eine optische Durchführung am Implantat existiert, die die optischen Signale in das Implantatsinnere leitet.

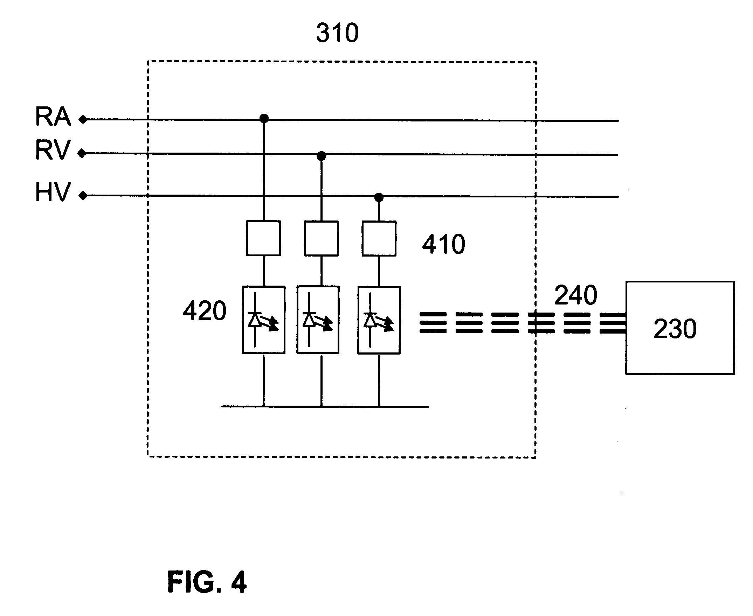

Die in Figur 4 gezeigt Ausführungsform bezieht sich auf einen Zweikammerschrittmacher mit Defibrillator. Die dazu notwendigen Leitungen sind mit RV (rechtes Ventrikel), RA (rechtes Atrium) und HV für die Schockelektroden gekennzeichnet, die mit der Eingangsschutzschaltung (310) verbunden sind. Die Eingangsschutzschaltung beinhaltet mehrere Schutzelemente, bevorzugt sind in diesem Fall drei Schutzelemente (410), also ein Schutzelement (410) für jede Elektrodenleitung (RV, RA und HV), die jeweils mit einer Elektrodenleitung verbunden sind, und mehrer elektrooptische Wandler (420), bevorzugt sind in diesem Fall drei elektrooptische Wandler (420) die jeweils mit einem Schutzelement (410) verbunden sind. Zusätzlich führen drei Lichtleiter (240) von jeweils einem elektrooptischen Wandler (420) zu einer Steuereinheit (230). Vorteil dieser Ausführung ist neben anderen, dass die Steuereinheit (230) auf die unterschiedlichen Signale auf den einzelnen Elektrodenleitungen (RA, RV und HV) unterschiedlich reagieren kann und auch unterschiedliche Modi von der in Figur 4 nicht gezeigten Implantatssteuerung (220) ausgewählt werden können. Die unterschiedlichen Modi können bevorzugt unterschiedliche Maßnahmen für aller oder einzelne Elektrodenleitungen und/oder das gesamte Implantat und/oder einzelne Bestandteile des Implantats bewirken.

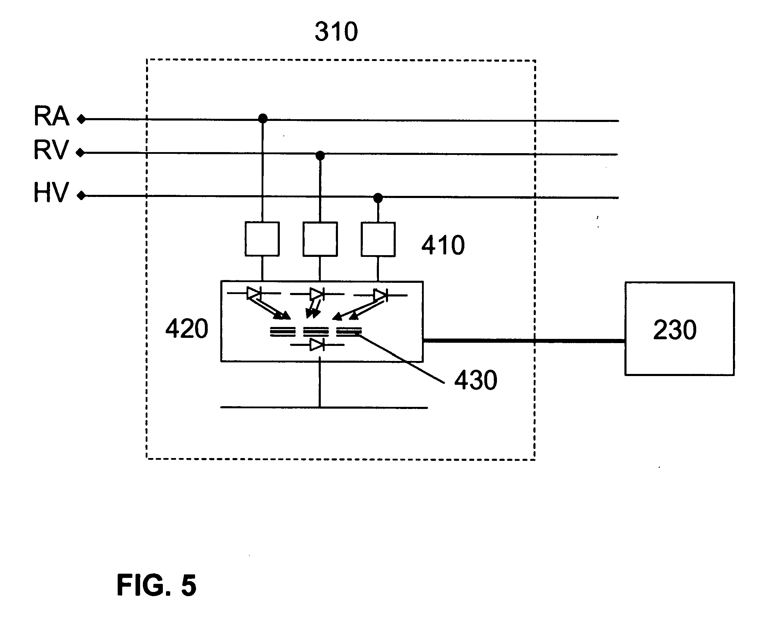

Die in Figur 5 gezeigte Ausführung ist bis auf einen Unterschied in der Übertragung der optischen Signale analog zu der in Figur 4 aufgebaut. Der wesentliche Unterschied besteht darin, dass die elektrooptischen Wandler (420) zusätzlich jeweils einen Gewichtungsfilter (430) aufweisen. Die lichtemittierenden Elemente (LEDs) strahlen auf ein und denselben Sensor. Dies entspricht einer ODER-Verknüpfung. Um die Wichtigkeit der einzelnen Beiträge verschiedener Implantatseingänge hinsichtlich der eingekoppelten Störung zu berücksichtigen, erfolgt eine Gewichtung der optischen Signale. Dies erfolgt durch einen optischen Filter, dessen Dämpfung, für jedes lichtemittierende Element getrennt einstellbar, bezogen auf die Wichtigkeit einzelnen Eingänge die Gewichtungsfaktoren realisiert.

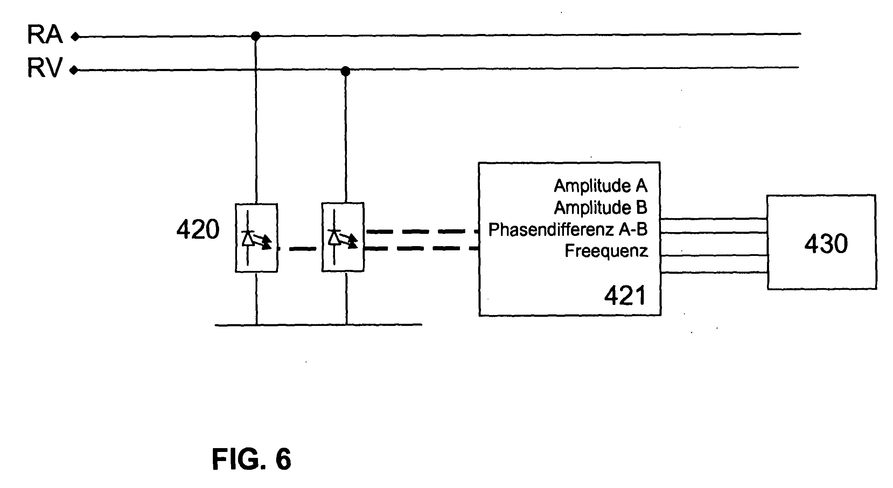

In einer weiteren Ausführung, die in Figur 6 skizziert ist, werden die detektierten und induzierten Signale, wie bei den vorherigen Beispielen, von den Elektrodenleitungen, hier sind der Einfachheit halber nur zwei Elektrodenleitungen (RA und RV) aufgeführt, es können aber auch mehr sein, an jeweils einen elektrooptischen Wandler (420) weitergeleitet. Die vom elektrooptischen Wandler emittierten optischen Signale werden dann über einen optischen Leiter zu einem weiteren elektrooptischen Wandler (421) geleitet, der aus dem Signal Amplituden und/oder Frequenzen und/oder Phasenbeziehungen ermittelt und diese an die Steuereinheit (430) weiterleitet. Die von dem zweiten elektrooptischen Wandler ermittelten Daten werden von der Steuereinheit mittels eines Schwellwertkomparators ausgewertet und das Ergebnis analog zu der Verfahrensweise in Figur 2 weiter verarbeitet.

Die genannten Ausführungen ermöglichen, dass das Vorhandensein eines MRT oder anderer hochfrequenter starker elektromagnetischer Felder, z. B. Patienten im Umfeld von Sendeanlagen, wie Mobilfunk Relaisstation, sicher von einem elektronischen Implantat erkannt werden kann, so dass das Implantat in einen (MRT-)sicheren Zustand umschalten kann. Die Erfindung soll dabei sicherstellen, dass die durch das MRT hervorgerufene RF-Störung nicht oder nur minimal in die elektronische Schaltung des Implantates eingekoppelt werden kann.

## Patentansprüche

1. Implantierbares medizinisches Gerät (IMD) mit einem hermetisch abgeschlossenen Gehäuse, mindestens einer Steuereinheit, mindestens einer Erkennungseinheit für MRT-Störfelder, die mit mindestens der einen Steuereinheit und mindestens einer Elektrode und/oder mindestens einer Antenne und/oder mindestens einer Spule verbunden oder verbindbar ist, **dadurch gekennzeichnet, dass**
die Erkennungseinheit für MRT-Störfelder mindestens einen elektrooptischen Wandler beinhaltet, der induzierte Spannungen aus der mindestens einen Elektrode und/oder der mindestens einen Antenne und/oder der mindestens einen Spule in optische Signale umwandelt und diese innerhalb der Erkennungseinheit für MRT-Störfelder an eine Auswerteeinheit der Erkennungseinheit für MRT-Störfelder optisch und potentialfrei überträgt und die Auswerteeinheit bei Überschreiten einer Schwelle der optischen signale und/oder einem vorbestimmten periodischen Auftreten der optischen Signale ein Umschalten in einen MRI-sicheren Zustand auslöst.

2. IMD nach Anspruch 1, **dadurch gekennzeichnet, dass** die Erkennungseinheit für MRT-Störfelder über mindestens einen Sensor verfügt, der die optischen Signale von dem mindestens einen elektrooptischen Wandler in elektrische Signale umwandelt, die zur MRT-Erkennung verwendet werden, wobei auch die Signale von mehreren elektrooptische Wandler zu einem Sensor führen können.

3. IMD nach einem der vorhergegangenen Ansprüche, **dadurch gekennzeichnet, dass** mindestens eine Elektrode vorhanden ist, die therapeutischen und/oder diagnostischen Zwecken dient, wobei die Elektrode sich außerhalb der des hermetisch verschlossen Gehäuses erstrecken kann oder sich auf der Außenfläche diese Gehäuses befinden kann oder Teil dieses Gehäuses sein kann.

4. IMD nach Anspruch 3, **dadurch gekennzeichnet, dass** die mindestens eine Elektrode eine Verbindung zu mindestens einem, vom Implantat ausgehenden distal angeordneten Sensor und/oder mindestens einem Aktuator ist.

5. IMD nach einem der vorangegangenen Ansprüche, **dadurch gekennzeichnet, dass** der elektrooptische Wandler ein Optokoppler ist.

6. IMD nach einem der vorangegangenen Ansprüche, **dadurch gekennzeichnet, dass** der elektrooptische Wandler ein Sensor für elektromagnetische Felder ist, der auf den von externen elektromagnetischen Feldern abhängigen optischen Eigenschaften eines Kristalls basiert.

7. IMD nach einem der vorangegangenen Ansprüche, **dadurch gekennzeichnet, dass** der elektrooptische Wandler nur Informationen zu:
- den Amplituden und/oder
- Einhüllende der Amplituden und/oder
- den Frequenzen und/oder
- bei mehren elektrooptischen Wandlern- Phasen überträgt

8. IMD nach Anspruch 7, **dadurch gekennzeichnet, dass** die Erkennungseinheit für MRT-Störfelder aus dem optischen Signal einen skalaren Wert ermittelt, der die identifizierte Feldstärke repräsentiert und bei Überschreiten eines voreinstellbaren und/oder variablen Schwellwertes oder bei Erreichen eines voreinstellbaren und/oder variablen Schwellwertbereichs ein MRT-Feld erkennt, wobei der skalare Wert auch eine funktionale oder logische Verknüpfung aus einer oder mehreren der Informationen, wie Amplitudeninformation und/oder Frequenzinformation und/oder Phaseninformation, oder der gewichteten Informationen sein kann.

9. IMD nach Anspruch 8, **dadurch gekennzeichnet, dass** die Gewichtung der Informationen über eine vorbestimmte Dämpfung der optischen Signale bei der Übertragung der optischen Signale erfolgt, wobei zusätzlich unterschiedliche Dämpfungen für unterschiedlichen Signalquellen oder Informationsarten über verschiedene Übertragungsfrequenzen realisiert werden können.

10. IMD nach einem der vorangegangenen Ansprüche, **dadurch gekennzeichnet, dass** mehrere elektrische Zuleitungen zum elektrooptischen Wandler in Reihe und/oder parallel geschaltet sind.

11. IMD nach einem der vorangegangenen Ansprüche, **dadurch gekennzeichnet, dass** der elektrooptische Wandler mit mindestens einem Schutzelement in Reihe und/oder parallel geschaltet ist.

12. IMD nach einem der vorangegangenen Ansprüche, **dadurch gekennzeichnet, dass** der Schwellwert zur MRT-Erkennung eine gewichtete Funktion der ermittelten Frequenz der induzierten Spannungen ist und/oder der Schwellwert und/oder die Gewichtungsfaktoren eine Funktion von mit anderen Sensoren oder Indikatoren bestimmten statischen Feldstärke ist oder sind, wobei die funktionale Abhängigkeit sowohl linear als auch nicht linear sein kann.

13. IMD nach einem der vorangegangenen Ansprüche, **dadurch gekennzeichnet, dass** der durch die MRT-Störerkennungseinheit eingeschaltete oder ausgelöste MRT-sichere Zustand für eine vorbestimmte oder vorbestimmbare Zeit eingeschaltet und nach dem Ablauf der Zeit deaktiviert oder eine weitere MRT-Erkennung vorgenommen.

14. IMD nach einem der vorangegangenen Ansprüche, **dadurch gekennzeichnet, dass** eine MRT-Erkennung nur stattfindet, wenn zeitgleich mit der MRT-Erkennung nach Anspruch 1 auch mindestens eine weitere Messmethode eine MRT-Erkennung signalisiert, wobei unter weiteren Methoden unter anderem, aber nicht ausschließlich, folgende Methoden zu verstehen sind:
GMR-Sensoren,
MagFET-Sensoren,
Hallsensoren,
die Überwachung von Batteriespannungen während Kondensatorladeprozessen,
die Detektion von RF-Feldern,
die Detektion von magnetischen Grandientenfeldern,
die Detektion von durch elektromagnetische Felder induzierten Strömen,
die Detektion von spezifischen Vibrationen oder als Sensoren ausgelegte Bauteile zur Detektion von durch Lorentzkräfte induzierten Vibrationen.

15. IMD nach einem der vorangegangenen Ansprüche, **dadurch gekennzeichnet, dass** mindestens eine der folgenden Maßnahmen bei einer MRT-Erkennung oder durch das MRT-Erkennungssignal eingeleitet werden:
- das Wechseln in einen MRI-Sicherenzustand
- ein verlängertes Verbleiben in einem MRI-sicheren oder gegenüber elektromagnetischen Störfeldern unempfindlichen Zustand,
- eine Synchronisierung von elektrischen Messungen (z. B. Impedanzmessungen) mit bei periodischen oder gepulsten elektromagnetischen Feldern auftretenden betragsmäßigen Feldstärkenminima oder die Synchronisierung einer Stimulation mit eben jenen betragsmäßigen Minima, und
- die Abgabe von elektromagnetischen Pulsen zur Signalisierung, dass ein medizinisches Gerät, speziell ein Implantat, im elektromagnetischen Feld vorhanden ist, speziell zur Signalisierung an ein MRI-Gerät, mit der Möglichkeit neben der Störung auch Informationen auf diese Weise zu übertragen und auf dem Bildschirm des MRI sichtbar zu machen.

## Claims

1. An implantable medical device (IMD) with a hermetically closed housing, at least one control unit, at least one unit for identifying MRT (magnetic resonance tomography) interference fields, which is connected or can be connected at least to the control unit and at least one electrode and/or at least one antenna and/or at least one coil, **characterised in that**
the unit for identifying MRT interference fields contains at least one electro-optical converter, which converts induced voltages from the at least one electrode and/or the at least one antenna and/or the at least one coil into optical signals and, within the unit for identifying MRT interference fields, forwards these signals optically and in a potential-free manner to an evaluation unit of the unit for identifying MRT interference fields, and the evaluation unit triggers a switchover into an MRI-safe (magnetic-resonance-imaging-safe) state when a threshold of the optical signals and/or a predetermined periodic occurrence of the optical signals is exceeded.

2. The IMD according to Claim 1, **characterised in that** the unit for identifying MRT interference fields has at least one sensor, which converts the optical signals from the at least one electro-optical converter into electrical signals, which are used for MRT identification, wherein the signals from a plurality of electro-optical converters may also lead to a sensor.

3. The IMD according to one of the preceding claims, **characterised in that** at least one electrode is provided, which is used for therapeutic and/or diagnostic purposes, wherein the electrode may extend outside the hermetically sealed housing or may be located on the outer face of this housing or may be part of this housing.

4. The IMD according to Claim 3, **characterised in that** the at least one electrode is a connection to at least one sensor arranged distally starting from the implant and/or at least one actuator.

5. The IMD according to one of the preceding claims, **characterised in that** the electro-optical converter is an optocoupler.

6. The IMD according to one of the preceding claims, **characterised in that** the electro-optical converter is a sensor for electromagnetic fields, which is based on the optical properties, dependent on external electromagnetic fields, of a crystal.

7. The IMD according to one of the preceding claims, **characterised in that** the electro-optical converter only forwards information concerning:
- the amplitudes and/or
- envelopes of the amplitudes and/or
- the frequencies and/or
- phases, in configurations with multiple electro-optical converters.

8. The IMD according to Claim 7, **characterised in that** the unit for identifying MRT interference fields determines from the optical signal a scalar value, which represents the identified field strength and, when a presettable and/or variable threshold value is exceeded or when a presettable and/or variable threshold value range is reached, identifies an MRT field, wherein the scalar value may also be a functional or logical link formed from one or more pieces of information, such as amplitude information and/or frequency information and/or phase information, or weighted information.

9. The IMD according to Claim 8, **characterised in that** the information is weighted via a predetermined damping of the optical signals during the transmission of the optical signals, wherein additional different dampings can be implemented for different signal sources or types of information over various transmission frequencies.

10. The IMD according to one of the preceding claims, **characterised in that** a plurality of electrical supply lines to the electro-optical converter are connected in series and/or in parallel.

11. The IMD according to one of the preceding claims, **characterised in that** the electro-optical converter is connected to at least one protective element in series and/or in parallel.

12. The IMD according to one of the preceding claims, **characterised in that** the threshold value for MRT identification is a weighted function of the determined frequency of the induced voltages and/or the threshold value and/or the weighting factors is/are a function of static field strengths determined via other sensors or indicators, wherein the functional dependency may be either linear or non-linear.

13. The IMD according to one of the preceding claims, **characterised in that** the MRT-safe state switched on or triggered by the MRT interference identification unit is switched on for a predetermined or predeterminable time and, once this time has elapsed, said MRT-safe state is deactivated or a further MRT identification process is performed.

14. The IMD according to one of the preceding claims, **characterised in that** MRT identification only takes place if, at the same time as the MRT identification according to Claim 1, at least one further measurement method also signals MRT identification, wherein further methods are to be understood, *inter alia* but not exclusively, to be the following methods:
GMR sensors,
MagFET sensors,
Hall sensors,
the monitoring of battery voltages during capacitor charging processes,
the detection of RF fields,
the detection of magnetic gradient fields,
the detection of currents induced by electromagnetic fields,
the detection of specific vibrations or components designed as sensors for the detection of vibrations induced by Lorentz forces.

15. The IMD according to one of the preceding claims, **characterised in that** at least one of the following measures is initiated with MRT identification or by the MRT identification signal:
- change to an MRI-safe state,
- remain for a prolonged period of time in an MRI-safe state or a state that is insensitive to electromagnetic interference fields,
- synchronise electrical measurements (for example impedance measurements) with use of field strength minimum values that occur with periodic or pulsed electromagnetic fields, or synchronise a stimulation with use of said field strength minimum values, and
- emit electromagnetic pulses to signal (specifically to an MRI device) that a medical device, specifically an implant, is present in the electromagnetic field, with the possibility of also transferring information in this way, in addition to the interference, and displaying this on the screen of the MRI device.

## Revendications

1. Dispositif médical implantable (IMD) avec un boîtier fermé hermétiquement, au moins une unité de commande, au moins une unité de reconnaissance pour des champs électriques parasites d'IRM, reliée ou reliable à l'au moins une unité de commande et à au moins une électrode et/ou au moins une antenne et/ou au moins une bobine, **caractérisé en ce que**
l'unité de reconnaissance pour des champs électriques parasites d'IRM contient un convertisseur électro-optique convertissant les tensions induites venant de l'au moins une électrode, et/ou de l'au moins une antenne et/ou de l'au moins une bobine, en signaux optiques et transmettant ceux-ci dans l'unité de reconnaissance pour des champs électriques parasites d'IRM à une unité d'évaluation de l'unité de reconnaissance pour des champs électriques parasites d'IRM, de façon optique et sans potentiel, et l'unité d'évaluation déclenche le passage à un état fiable d'IRM, lorsque les signaux optiques dépassent un certain seuil et/ou une production périodique prédéterminée de signaux optiques.

2. Dispositif médical implantable selon la revendication 1, **caractérisé en ce que** l'unité de reconnaissance pour des champs électriques parasites d'IRM dispose d'au moins un capteur convertissant les signaux optiques de l'au moins un convertisseur électro-optique en signaux électriques, lesquels sont utilisés pour la reconnaissance IRM, les signaux de plusieurs convertisseurs électro-optiques pouvant mener à un même capteur.

3. Dispositif médical implantable selon l'une des revendications précédentes, **caractérisé en ce qu'**il est prévu au moins une électrode servant à des fins thérapeutiques et/ou pour le diagnostic, l'électrode pouvant s'étendre à l'extérieur du boîtier fermé hermétiquement et se trouver sur la surface extérieure de ce boîtier ou encore faire partie de ce boîtier.

4. Dispositif médical implantable selon la revendication 3, **caractérisé en ce que** l'au moins une électrode établit une liaison vers au moins un capteur agencé de façon distale par rapport à l'implant, et/ou vers au moins un actionneur.

5. Dispositif médical implantable selon l'une des revendications précédentes, **caractérisé en ce que** le convertisseur électro-optique est un coupleur optique.

6. Dispositif médical implantable selon l'une des revendications précédentes, **caractérisé en ce que** le convertisseur électro-optique est un capteur pour champs électromagnétiques, lequel est formé à base des propriétés optiques d'un cristal, **en ce que** les propriétés optiques sont dépendantes des champs électriques externes.

7. Dispositif médical implantable selon l'une des revendications précédentes, **caractérisé en ce que** le convertisseur électro-optique transmet seulement des informations concernant :
- les amplitudes et/ou
- des enveloppes d'amplitudes et/ou
- les fréquences et/ou
- des phases, en présence de plusieurs convertisseurs électro-optiques.

8. Dispositif médical implantable selon la revendication 7, **caractérisé en ce qu'**à partir du signal optique, l'unité de reconnaissance pour des champs électriques parasites d'IRM détermine une grandeur scalaire représentant l'intensité de champ, et reconnaît un champ IRM lors du dépassement d'une valeur seuil variable et/ou préréglable ou lors de l'attente d'une certaine plage de valeurs seuil variable et/ou préréglable, la grandeur scalaire pouvant également être une combinaison logique ou fonctionnelle à partir d'une ou de plusieurs des informations, telles qu'une information d'amplitude et/ou une information de fréquence et/ou une information de phase, ou des informations pondérées.

9. Dispositif médical implantable selon la revendication 8, **caractérisé en ce que** la pondération des informations est réalisée par une atténuation prédéterminée des signaux optiques lors de la transmission des signaux optiques, sachant qu'il est en outre possible de réaliser différentes atténuations pour différentes sources de signaux ou types d'informations, avec différentes fréquences de transmission.

10. Dispositif médical implantable selon l'une des revendications précédentes, **caractérisé en ce que** plusieurs lignes d'alimentation électriques vers le convertisseur électro-optique sont connectées en série et/ou en parallèle.

11. Dispositif médical implantable selon l'une des revendications précédentes, **caractérisé en ce que** le convertisseur électro-optique est connecté en série et/ou en parallèle avec au moins un élément de protection.

12. Dispositif médical implantable selon l'une des revendications précédentes, **caractérisé en ce que** la valeur seuil pour la reconnaissance IRM est une fonction pondérée de la fréquence déterminée pour les tensions induites, et/ou la valeur seuil et/ou les facteurs de pondération est/sont une fonction de l'intensité de champ statique déterminée avec d'autres capteurs ou indicateurs, la dépendance fonctionnelle pouvant être aussi bien linéaire que non linéaire.

13. Dispositif médical implantable selon l'une des revendications précédentes, **caractérisé en ce que** l'état fiable d'IRM déclenché ou activé par l'unité de reconnaissance de champs électriques parasites d'IRM est activé pour une durée prédéterminée ou prédéterminable, puis désactivé après l'écoulement de la durée, ou une nouvelle reconnaissance IRM est effectuée.

14. Dispositif médical implantable selon l'une des revendications précédentes, **caractérisé en ce que** la reconnaissance IRM n'est effectuée que si, conjointement avec la reconnaissance IRM selon la revendication 1, au moins un autre procédé de mesure signalise également une reconnaissance IRM, les autres procédés incluant entre autres, mais sans limitation, les procédés suivants :
capteurs GMR
capteurs MagFET
capteurs Hall
la surveillance des tensions de batterie pendant les opérations de chargement de condensateur,
la détection de champs de gradient magnétiques,
la détection de flux induits par des champs électromagnétiques,
la détection de vibrations spécifiques ou de pièces conçues comme des capteurs,
pour la détection de vibrations induites par des forces de Lorentz.

15. Dispositif médical implantable selon l'une des revendications précédentes, **caractérisé en ce qu'**au moins l'une des mesures suivantes est lancée lors de la reconnaissance IRM ou par le signal de reconnaissance IRM :
- le passage à un état fiable d'IRM
- la prolongation d'un état fiable d'IRM ou non sensible aux champs électromagnétiques parasites,
- une synchronisation de mesures électriques (par exemple des mesures d'impédance) avec des minima d'intensité de champ produits en cas de champs magnétiques périodiques ou pulsés, ou la synchronisation d'une stimulation avec lesdits minima, et
- l'émission d'impulsions électromagnétiques pour signaler la présence d'un dispositif médical, en particulier d'un implant, dans le champ électromagnétique, en particulier pour informer un dispositif IRM, avec la possibilité, de transmettre également des informations, en plus des parasites, et de les rendre visibles sur l'écran de l'IRM.
